# EUROPEAN PATENT APPLICATION

(11) **EP 3 673 792 A1**
(43) Date of publication of application: **01.07.2020**
(21) Application number: 19000568.6
(22) Date of filing: 19.12.2019
(51) Int. Cl.: A61B 5/00, A61B 5/053, A61B 5/18, A61B 5/16

(54) **A DEVICE FOR CARRYING OUT PSYCHOLOGICAL TESTS AS PART OF PROFESSIONAL SUITABILITY AND QUALIFICATION FOR WORK VERIFICATION PROCESS**

(30) Priority: 27.12.2018 PL 42837018
(71) Applicant: Politechnika Slaska, 44-100 Gliwice (PL)
(72) Inventor: Dobrowolska, Malgorzata, 40-540 Katowice (PL); Gzik,Marek, 40-100 Gliwice (PL); Wodarski, Piotr, 43-190 Mikolow (PL); Jedrasiak, Karol, 44-190 Mikolow (PL); Nawrat, Aleksander, 41-807 Zabrze (PL); Szabo, Stanislav, 044 41 Sady Nad Torysou (SK); Rozenberg, Robert, 04001 Kosice (SK)

(57) **Abstract**

A device for carrying out psychological tests as part of professional suitability and qualification for work verification process, constructed in a form of a tube (2) on a base (4) with control elements and sensors placed thereon, characterized in that it consists of a compartment (1) in a form of a tube closed with a door (2) so that to provide light and air separation from the outside environment. In the door (2) attached are lighting elements (13). On the floor (4) there is located an inner wall (9) with located thereon a panel (5) with control elements in a form of press buttons (10) and a display (12) and mobile elements (11) for manual exercises, on the panel (5) above the press buttons (10) there is the liquid-crystal touch display (12), mobile elements (11) for manual exercises are located in a side part of the panel (5) on the right and left side, on the opposite side of the panel (5) there is an element (3) in a form of a support with located thereon at least four skin conductivity sensors (6), on the upper part of the apparatus (1) there is an element (7) allowing setting of relevant air flow through the tube, on the upper part of the apparatus (1) there is an element (8) securing relevant humidity inside the apparatus

## Description

The object of the invention is a device for carrying out psychological tests as part of professional suitability and qualification for work verification process.

Psychological tests performed in relation to suitability for a job and qualifications for a job require a candidate to present relevant professional qualities related to sensory and motor abilities. That form of assessment may be performed with the use of special methods and apparatus.

Legal regulations indicate industrial occupations classified as so called difficult and hazardous occupations, health and life-threatening, requiring particular psycho-physical skills, subject to compulsory testing within the framework of occupational medicine, among others psychological testing.

An example of high risk occupation in the industry are the jobs involving work at chemical installations for production of toxic gases or gases forming explosive mixtures with air; work at open radioactive sources of class I and II; drill operator work and basic machinery operator work in opencast mining; work related to site clearing of explosive objects in underground mining facilities; work related to operation and movement maintenance of nuclear reactors, accelerators, generators, neutrons, chambers for production of radioactive sources, etc..

Having the above in mind, in order to perform psychological assessment, it is necessary to use apparatus allowing assessment of psycho-physical potential.
The present apparatus has been developed to measure human sensory and motor ability. Sensory and motor abilities include: visual acuity, colour vision, stereoscopic vision, mesopic vision, visual and motor coordination, fast reaction, perceptiveness, hand dexterity, hearing acuity, sense of balance, sense of touch.

The apparatus has been developed to measure, analyse and assess body reaction to stimuli perceived by human senses. The apparatus is able to generate the stimuli: visual, aural, tactile. The measurement includes strength of a stimulus, categorisation, scaling according to selected features. The apparatus measures time of reaction to the stimulus (medium, maximum, minimum), a number of correct, omitted and incorrect reactions to the stimulus, as well as a level of stress of the participant based on skin conductance measurement. Depending on the needs, it is possible to set a rate for the apparatus, a number of stimuli per minute or test duration and a number of stimuli. A task for the participant is to react to the stimuli by: pressing, pulling, moving a knob, touching when the light is on as fast as possible, in the constant time or the time manually adjusted by the psychologist. The light is a tri-colour one and may be red, green or blue.

A similar solution, "Cabine de stimulation sensorielle", known from patent description no. FR2705033A1, presents a closed cabin for sensory stimulation together with a possibility to modify light intensity and with control elements in a form of a panel with push buttons. However, the solution is limited to stimulate senses of the participant. The reaction is not measured, it does not include mobile elements in a form of manipulators and it does not allow to modify the humidity in the room. The apparatus "Apparatus for monitoring a person's psycho-physiological condition" known from patent description no. US6067468, is developed to perform psychological tests and ability tests. It includes a controller in a form of elements placed on hands and a panel with push buttons as well as a display in a form of a monitor. Similarly, a solution "Interactive psycho-physiological profiler method and system" known from patent description no. US20100292545A1, serves to assess a level of stress of the participant on the basis of sensors placed on the patient during testing. The testing is performed at a specially designed position. That solution is adapted to operate in an open space and does not allow to modify lighting when testing. A similar apparatus "System and procedure for estimation of psychological state based on psycho-physiological responses and transmission of the estimated state over various networks" known from patent description no. WO2010104480A1 only allows assessment of a psychological condition of the participant and not testing their manual abilities at modified lighting in a closed space.

There is no apparatus to perform psychological testing with respect to suitability for a job and qualifications for a job with simultaneously performed measurement of a vector of pressing force, pulling force, torque, reaction to a stimulus, and a level of stress of the participant. The present solution allows to reduce the time of testing in comparison to currently used research methods. The apparatus combines the advantages and functionalities of plurality of the research methods being used into one system, which reduces the costs to be incurred in case of construction of a psychological research and testing office.

The subject of the invention is a device for carrying out psychological tests as part of professional suitability and qualification for work verification process in a form of a tube which may be inclined at any angle for the need of testing performed in specific conditions. The apparatus is being closed to perform the test in particular lighting conditions or at no lighting, as well as in particular humidity secured by a humidity control element and at particular air circulation secured by a ventilation element. The apparatus comprises a place to locate the participant, sensors to measure skin conductivity and a panel with manual elements. Due to the use of the tube where the participant enters and a possibility of upright standing, as well as a change of the position, it allows measurement of visual and motor coordination, perceptiveness, concentration and shift of attention for occupations with different types of tasks. The participant has to make a decision under time pressure which buttons and what actions to take: press, pull, turn in, touch, etc.. The testing is performed at different options: at any pace, the programme emits a specified number of stimuli in the pace depending on the correct performance of the participant or at a pace imposed by the programme, the programme emits stimuli at a defined pace while the set of lights changes independently to the correct performance of the task. The test performance indicators are all the parameters, for example visual and motor coordination at any pace: duration of the performed task and a number of mistakes made, at an imposed pace: a number of received correct stimuli/reactions. The objective of mesopic vision testing is to establish a minimum light sensitivity freshhold. The participant is placed in the "tube" and upon adaptation to darkness provided is the presentation of visual stimuli which intensity is being gradually increased. The objective of the evaluation of the glare sensitivity is to provide information about the speed of adaptation of vision at darkness directly after the glare. The skin conductivity sensors placed in the apparatus allow measurement of skin-galvanic response, which is controlled by the sympathetic system when experiencing emotions. Such a phenomenon is typically used, for example, in lies detection.

The object of the invention is presented in the preferred embodiment in a drawing where Fig. 1 shows a cross-section view of the apparatus from the front, Fig. 2 shows a cross-section view of the apparatus from the side, Fig. 3 shows an axonometric view, Fig. 4 an axonometric view from another plane, and Fig. 5 a view of a panel with control elements.

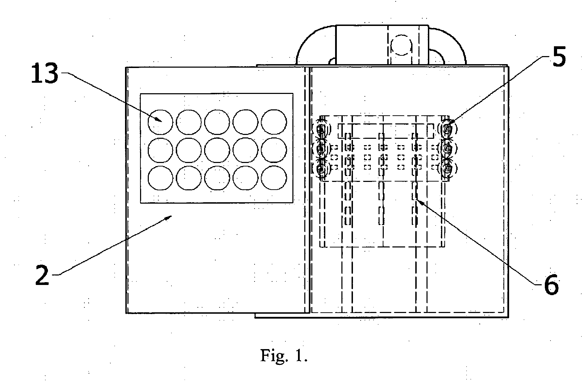

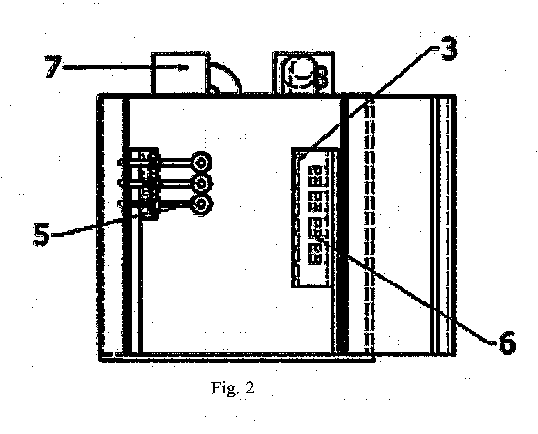

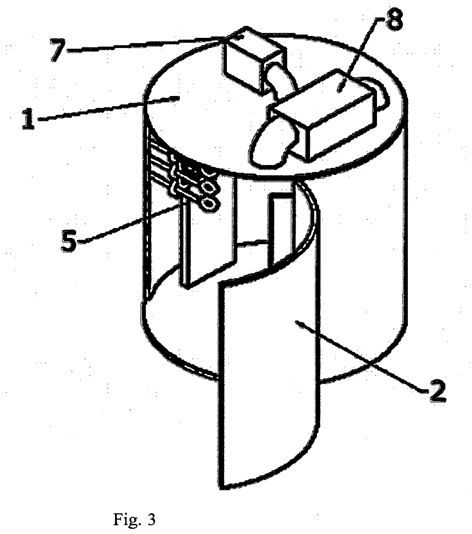

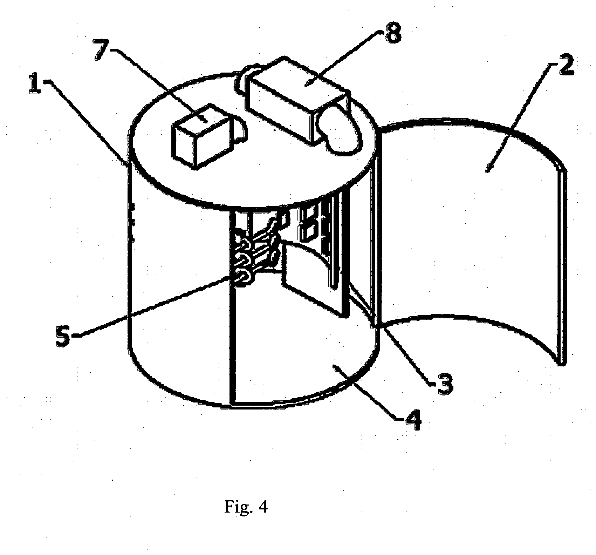

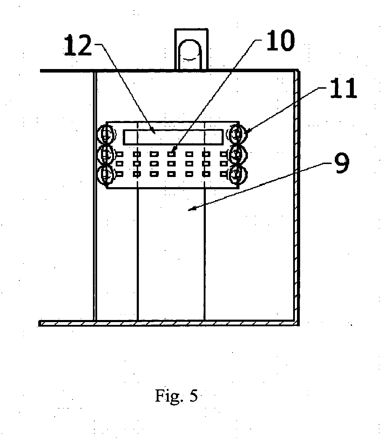

The present solution according to Figures 1, 2, 3, 4 and 5 comprises a compartment 1 in a form of a tube which may operate at any angle (at any inclination). The tube compartment 1 is being closed with a door 2 so that to provide light and air separation from the outside environment. In the door 2 attached are lighting elements 13 which allow modification of lighting intensity and colour with at least 3 colours: red, green, blue, and as a result modification of a level of the lighting of the apparatus interior. In the compartment 1 on a floor 4 is located an inner wall 9 with a panel with control elements 5 attached thereto in a form of press buttons 10 and a display 12 and with mobile elements 11 for manual exercises. The wall 9 allows adjustment of height of the panel with control elements 5. The panel 5 with the elements in a form of the press buttons 10 comprises at least twenty one lit with at least 3 colours illuminated press buttons 10 placed in at least seven columns and at least three rows. On the panel 5 above the press buttons 10 there is a liquid-crystal touch display 12. The mobile elements 11 for manual exercises are placed on a side part of the panel 5 at the right and left side. The apparatus comprises at least six mobile elements 11 allowing pulling, pushing and rotation. The mobile elements 11 are constructed in a form of a manipulator with three degrees of freedom. For all movements there is a possibility to set resistance force or torque. At the opposite side of the panel 5 there is an element 3 in a form of a support with located thereon at least four skin conductivity sensors 6 (measured in micro simens units µS). At the upper part of the apparatus 1 located is an element 7 allowing to set relevant air flow through the tube. This element is provided in a form of a ventilator with adjustable rotation speed and ventilator blades arrangement. At the upper part of the apparatus 1 there is also an element 8 securing relevant humidity inside the apparatus in the range from 10% to 95%.

The participant rests upon the element 3 touching the sensors 6 which provide measurement of the skin conductivity during the testing. Based on the measurement a level of stress of the participant is being established. The participant moves the elements 11 and presses on the buttons 10 on the panel 5. The task content and test results are presented on the display 12 on the panel 5.

### Patent claims:

1. A device for carrying out psychological tests as part of professional suitability and qualification for work verification process constructed in a form of a tube (2) on a base (4) with control elements and sensors placed thereon, **characterised in that** it consists of a compartment (1) in a form of a tube closed with a door (2) so that to provide light and air separation from the outside environment. In the door (2) attached are lighting elements (13). On the floor (4) there is located an inner wall (9) with located thereon a panel (5) with control elements in a form of press buttons (10) and a display (12) and mobile elements (11) for manual exercises, on the panel (5) above the press buttons (10) there is the liquid-crystal touch display (12), mobile elements (11) for manual exercises are located in a side part of the panel (5) on the right and left side, on the opposite side of the panel (5) there is an element (3) in a form of a support with located thereon at least four skin conductivity sensors (6), on the upper part of the apparatus (1) there is an element (7) allowing setting of relevant air flow through the tube, on the upper part of the apparatus (1) there is an element (8) securing relevant humidity inside the apparatus.
2. The device according to claim 1, characterised in that the wall (9) allows height adjustment of the panel with the control elements (5).
3. The device according to claim 1, characterised in that the panel (5) with elements in a form of the press buttons (10) comprises at east twenty one illuminated with at least 3 colours press buttons (10) placed in at least seven columns and at least three rows.
4. The device according to claim 1, characterised in that in the door (2) mounted are at least fifteen lighting elements (13) which functionality allows modification of lighting intensity, and as a result modification of illumination of the apparatus interior.
5. The device according to claim 1, characterised in that the apparatus comprises at least six mobile elements (11) allowing pulling, pushing and rotation.
6. The device according to claim 1, characterised in that the mobile elements (11) are constructed in a form of a manipulator with three degrees of freedom.
7. The device according to claim 1, characterised in that for all movements of the mobile elements (11) there is a possibility to set resistance force or torque.
8. The device according to claim 1, characterised in that the element (7) comprises a ventilator with adjustable rotation speed and blades arrangement.
9. The device according to claim 1, characterised in that the element (8) secures relevant humidity inside the apparatus in the range from 10% to 95%.
10. The device according to claim 1, characterised in that the measurement of the skin conductivity is performed with the wired skin conductivity sensor (6).

## Claims

1. A device for carrying out psychological tests as part of professional suitability and qualification for work verification process constructed in a form of a tube (2) on a base (4) with control elements and sensors placed thereon, **characterised in that** it consists of a compartment (1) in a form of a tube closed with a door (2) so that to provide light and air separation from the outside environment. In the door (2) attached are lighting elements (13). On the floor (4) there is located an inner wall (9) with located thereon a panel (5) with control elements in a form of press buttons (10) and a display (12) and mobile elements (11) for manual exercises, on the panel (5) above the press buttons (10) there is the liquid-crystal touch display (12), mobile elements (11) for manual exercises are located in a side part of the panel (5) on the right and left side, on the opposite side of the panel (5) there is an element (3) in a form of a support with located thereon at least four skin conductivity sensors (6), on the upper part of the apparatus (1) there is an element (7) allowing setting of relevant air flow through the tube, on the upper part of the apparatus (1) there is an element (8) securing relevant humidity inside the apparatus.

2. The device according to claim 1, **characterised in that** the wall (9) allows height adjustment of the panel with the control elements (5).

3. The device according to claim 1, **characterised in that** the panel (5) with elements in a form of the press buttons (10) comprises at east twenty one illuminated with at least 3 colours press buttons (10) placed in at least seven columns and at least three rows.

4. The device according to claim 1, **characterised in that** in the door (2) mounted are at least fifteen lighting elements (13) which functionality allows modification of lighting intensity, and as a result modification of illumination of the apparatus interior.

5. The device according to claim 1, **characterised in that** the apparatus comprises at least six mobile elements (11) allowing pulling, pushing and rotation.

6. The device according to claim 1, **characterised in that** the mobile elements (11) are constructed in a form of a manipulator with three degrees of freedom.

7. The device according to claim 1, **characterised in that** for all movements of the mobile elements (11) there is a possibility to set resistance force or torque.

8. The device according to claim 1, **characterised in that** the element (7) comprises a ventilator with adjustable rotation speed and blades arrangement.

9. The device according to claim 1, **characterised in that** the element (8) secures relevant humidity inside the apparatus in the range from 10% to 95%.

10. The device according to claim 1, **characterised in that** the measurement of the skin conductivity is performed with the wired skin conductivity sensor (6).
